# EUROPEAN PATENT SPECIFICATION

(11) **EP 3 187 159 B1**
(45) Date of publication and mention of the grant of the patent: **05.07.2023**
(21) Application number: 17153636.0
(22) Date of filing: 20.08.2007
(51) Int. Cl.: A61F 9/08, A61F 9/008, A61F 2/14

(54) **SYSTEM FOR RESECTING CORNEAL TISSUE**
SYSTEM FÜR HORNHAUTGEWEBERESEKTION
SYSTÈME DE RÉSECTION DU TISSU CORNÉEN

(30) Priority: 05.09.2006 US 469899
(43) Date of publication of application: 05.07.2017
(62) Divisional of application: 07814263.5
(73) Proprietor: AMO Development LLC, Irvine, CA 92618 (US)
(72) Inventor: KURTZ, Ronald, M., Irvine, CA 92603 (US); JUHASZ, Tibor, Irvine, CA 92620 (US); SARAYBA, Melvin, A., Irvine, CA 92694 (US); STEINERT, Roger, Laguna Beach, CA 92651 (US)
(74) Representative: Carpmaels & Ransford LLP

(56) References cited:
- WO-A1-94/09849
- WO-A1-94/25107
- WO-A1-2004/017878
- DE-C1- 10 124 358
- US-B1- 6 325 792

## Description

### BACKGROUND OF THE INVENTION

The field of the present invention is systems and procedures for transplanting corneas.

A variety of techniques presently exist for performing corneal transplants. The tools used for the different techniques range from the traditional trephine to the more advanced laser surgical systems. Regardless of the technique or tool used for the transplant procedure, the overarching goal remains to provide the recipient with new corneal tissue while minimizing post-surgical complications such as induced astigmatism. Other desirable aspects of recent improvements include reducing the overall healing time and the likelihood of additional complications from wound ruptures.

One technique which attempts to use advanced laser surgical systems to help reduce post-surgical complications is disclosed in U.S. Patent No. 6,805,694. This technique relies on the theory that the inner and outer diameters of the undercut region should be chosen to have a specific ratio. Further, the theory postulates that the internal pressure of the eye will provide enhanced wound stability and reduce post-surgical complications by selection of an appropriate ratio. Unfortunately, when applied in actual transplant procedures, this theory does not rise up to meet expectations.

US 2006/0100612 describes a laser-based device for non-mechanical three-dimensional trepanation during cornea transplants. As shown in Figures 2 and 3 the donor cornea has a sawtoothed shaped raised areas along its rim whilst as shown in Figures 4 and 5 the donor cornea has a projection and as shown in Figures 6 and 7 the donor cornea has a radial denticulation for radial grooves, with in each case the recipient cornea having complementary features.

US 2002/0173779 describes a laser system for corneal grafting in which according to a preferred configuration the undercut is zig zag shaped.

### INSERT A

### BRIEF SUMMARY OF THE INVENTION

The present invention is set out in the appended claims. Described herein are a system and method for resecting corneal tissue as part of a transplant procedure, the method claimed being only performed on donor tissue from a donor cornea and not on tissue from a living human or animal. In the system, a surgical laser emits a pulsed laser beam which is directed into the cornea by a focusing assembly. An interface provides a plurality of incision patterns for selection of a sidecut pattern which includes an annular region. The selected sidecut pattern is received by a controller which employs the focusing assembly to move the focal point of the pulsed laser beam and incise a donor cornea according to the sidecut pattern, placing the incision corresponding to the annular region at a predetermined depth from the anterior corneal surface. The controller also employs the focusing assembly to move the focal point and incise a recipient cornea according to the sidecut pattern, again placing the incision corresponding to the annular region at the same predetermined depth from the anterior corneal surface.

In the method, the depth of an incision from the anterior corneal surface is initially determined. This incision depth is used for both the recipient cornea and the donor cornea, and in each cornea, an annular incision is made at the incision depth. A first sidecut incision is made running from the outer periphery of the annular incision toward one of the anterior corneal surface or the posterior corneal surface. A second sidecut incision is made running from the inner periphery of the annular incision toward the other of the anterior corneal surface or the posterior corneal surface. The angle of each sidecut incision to the annular incision may be acute, perpendicular, or obtuse. The combination of the incisions in each cornea resects corneal tissue from the recipient cornea and donor tissue from the donor cornea, respectively. The donor tissue is thereafter grafted into the recipient cornea. Following the graft, the donor tissue may be secured using a plurality of sutures, each preferably passing through the sidecut incision which is nearest the posterior surface of the recipient cornea.

The extent of the sidecut incisions depends upon whether the transplant is a full thickness corneal transplant or a partial thickness corneal transplant. In a full thickness transplant, each sidecut incision runs from the annular incision to one of the anterior corneal surface or the posterior corneal surface. A partial thickness transplant may be an anterior lamellar keratoplasty (ALK) or a posterior lamellar keratoplasty (PLK). In both procedures, a resection incision is made within the stromal tissue of each cornea. For the ALK procedure, the depth of the annular incision is between the anterior surface and the resection incision, whereas for the PLK procedure, the depth of the annular incision is between the posterior surface and the resection incision. Thus, in the ALK procedure, one of the sidecut incisions runs from the annular incision to the anterior surface and the other runs from the annular incision to the resection incision. Similarly, in the PLK procedure, one of the sidecut incisions runs from the annular incision to the posterior surface and the other runs from the annular incision to the resection incision.

Accordingly, an improved system for resecting corneal tissue and a method for resecting donor tissue from a donor cornea as part of a transplant procedure are disclosed. Advantages of the improvements will appear from the drawings and the description of the preferred embodiment.

### BRIEF DESCRIPTION OF THE DRAWINGS

In the drawings, wherein like reference numerals refer to similar components:

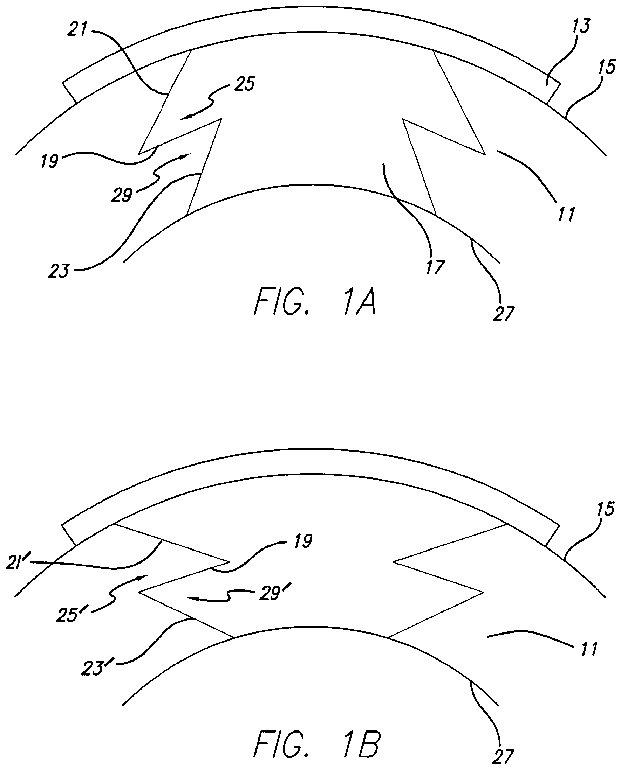

Fig. 1A & 1B illustrates a sectional view of a cornea with incisions made therein for a full thickness corneal tissue transplant;

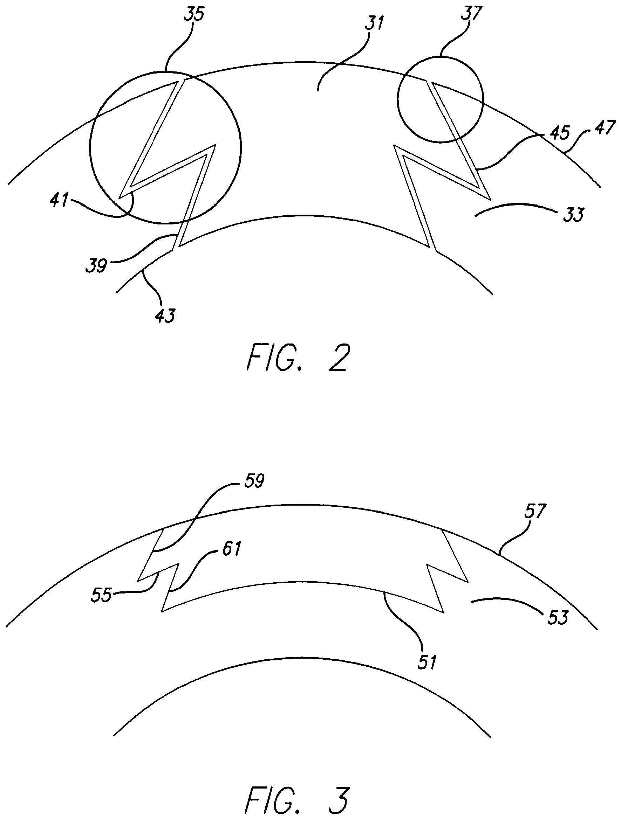

Fig. 2 illustrates a sectional view of donor tissue grafted into a recipient cornea;
Fig. 3 illustrates a sectional view of incisions made in a cornea for a partial thickness ALK corneal tissue transplant;

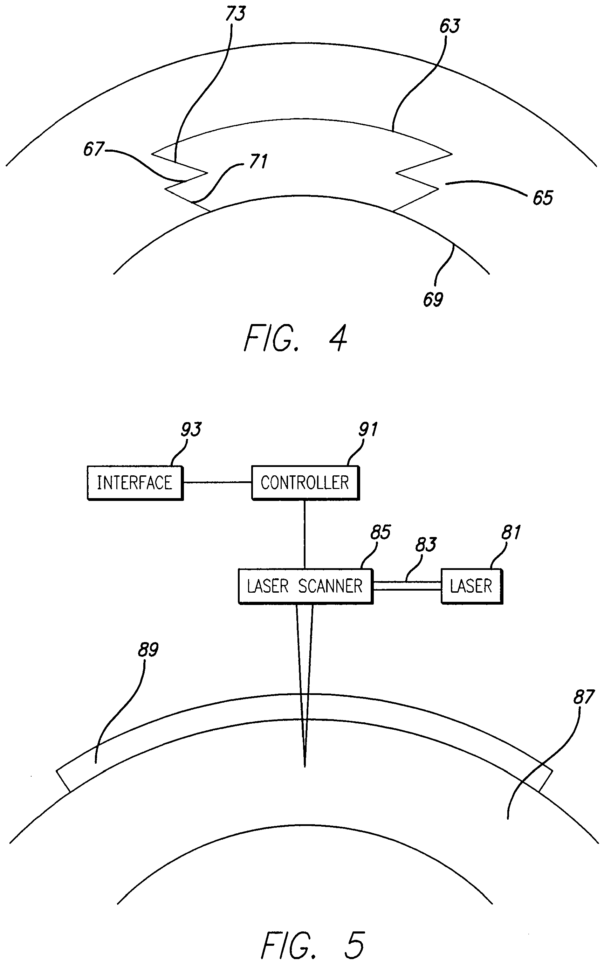

Fig. 4 illustrates a sectional view of incisions made in a cornea for a partial thickness PLK corneal tissue transplant; and
Fig. 5 is a schematic view of a system for resecting corneal tissue.

### DETAILED DESCRIPTION

Turning in detail to the drawings, Fig. 1A illustrates the incisions made in a cornea 11 as part of a full thickness corneal transplant procedure. The same incisions are made in both the recipient cornea and the donor cornea, although the incisions in the donor cornea may be made approximately 1%-5% larger, or more preferably 2%-5% larger, than the incisions in the recipient cornea to account for shrinkage in the donor corneal tissue following resection. As part of the procedure, a contact lens 13 placed against the anterior corneal surface 15. This contact lens 13 deforms the cornea 11, forcing the anterior corneal surface 15 to take on the shape of the contact lens 13. Deformation of the cornea 11 in this manner provides multiple advantages which are well known to skilled artisans. For example, U.S. Patent No. 5,549,632 describes advantages gained in making laser incisions by deforming the shape of the cornea, particularly by applanation. U.S. Patent No. 6,863,667 and U.S. Pat. App. No. 11/258,399,
describe patient interface devices which may be used to align the surgical laser with the recipient cornea for purposes of making accurate incisions.

Further, deformation of the cornea reduces the amount of physical data which needs to be collected for both the recipient cornea and the donor cornea prior to the transplant procedure. The physical data collected includes thickness measurements of both the recipient and donor corneas. These thickness measurements are used to develop a thickness profile of each cornea. Additional physical data may also be collected for each cornea. This thickness profile, along with any other data needed for the procedure, may be obtained by any one of the many known methods for measuring the physical structure of the eye, with the preferred method being through optical coherence tomography (OCT). Many commercially available OCT scanners are capable of performing such measurements. One example is the VisanteTM OCT scanning system, manufactured by Carl Zeiss Meditec, which has an office in Dublin, California. One advantage of the VisanteTM OCT system is that it does not make contact with the cornea when performing the OCT scan.

Three incisions, an annular incision 19 and two sidecut incisions 21, 23, are made in the cornea 11 to resect the corneal tissue 17. These incisions may be made separately, one at a time, or they may be made concurrently. The combined incisions result in corneal tissue being resected from the recipient cornea, and donor tissue being resected from the donor cornea. All incisions are preferably made using a pulsed laser beam having ultra-short pulses, preferably in the femtosecond range. The laser may be of the type described in U.S. Patent No. 4,764,930, producing an ultra-short pulsed beam as described in one or both of U.S. Patent No. 5,984,916 and U.S. Patent No. RE37,585. Commercial lasers capable of performing the incisions are available from IntraLase Corp. of Irvine, California.

The annular incision 19 is located at a predetermined depth from the anterior corneal surface 15. To facilitate the resection and grafting process, the entire annular incision 19 is at a uniform distance from the anterior corneal surface 15, although such uniformity of depth is not required. Further, for simplicity the annular incision 19 is described as being defined by an inner radius and an outer radius, although such is not necessary. It is sufficient for this incision to be formed by an inner perimeter and an outer perimeter, both perimeters being of any desired shape. More complex shapes, however, can add significantly to the complexity of the procedure. Various techniques are known for making the annular incision 19 at a uniform distance from the anterior corneal surface 15. For example, the techniques disclosed in U.S. Patent No. 5,993,438, U.S. Patent No. 6,730,074, and U.S. Patent Publication No. 20050245915 may be readily adapted to make the desired annular incision 19. Other techniques known to skilled artisans may also be employed.

The first sidecut incision 21 runs from the outer periphery of the annular incision 19 to the anterior corneal surface 15. An acute angle is formed at the juncture 25 of the first sidecut incision 21 and the annular incision 19. The second sidecut incision 23 runs from the inner periphery of the annular incision 19 to the posterior corneal surface 27. An acute angle is also formed at the juncture 29 of the second sidecut incision 23 and the annular incision 19. Alternatively, the angle between each of the sidecut incisions and the annular incision may be perpendicular or obtuse.

Fig. 1B illustrates an alternative configuration for the sidecut incisions 21', 23' made in the cornea 11 with respect to the annular incision 19. Here, the first sidecut incision 21' runs from the inner perimeter of the annular incision 19 to the anterior corneal surface 15, and the second sidecut incision 23' runs from the outer perimeter of the annular incision 19 to the posterior corneal surface 27.

The donor tissue 31 is shown grafted into the recipient cornea 33 in Fig 2. Sutures 35, 37 are placed to secure the donor tissue 31 to the recipient cornea 33. Two different techniques for placement of sutures are shown. The first suture 35 is placed through the sidecut incision 39 which runs between the annular incision 41 and the posterior corneal surface 43. The second suture 37 is placed through the sidecut incision 45 which runs between the annular incision 41 and the anterior corneal surface 47. Both locations of sutures are believed to be equally as effective for securing the donor tissue 31 to the recipient cornea 33. Further, neither location is believed to be more likely than the other to induce astigmatism during the healing process.

Fig. 3 illustrates how the incision configuration described above may be adapted for an ALK procedure. For the ALK process, a resection incision 51 is made in the cornea 53 at a predetermined resection depth, and the annular incision 55 is made at a depth which lies between the resection depth and the anterior corneal surface 57. The first sidecut incision 59 is made in the same manner as is described above. The second sidecut incision 61, however, runs from the annular incision to the resection incision 51. The donor tissue may be secured to the recipient cornea by either of the techniques described above.

Fig. 4 illustrates how the incision configuration described above may be adapted for an PLK procedure. For the PLK process, a resection incision 63 is made in the cornea 65 at a predetermined resection depth, and the annular incision 67 is made at a depth which lies between the resection depth and the posterior corneal surface 69. The first sidecut incision 71 is made in the same manner as is described above. The second sidecut incision 73, however, runs from the annular incision to the resection incision 63. In a PLK procedure, sutures are not necessary to secure the donor tissue to the recipient cornea.

Referring to Fig. 5, a surgical system is shown which may be used to incise both a donor cornea or a recipient cornea. Alternatively, two similarly configured systems may be used to incise each respective cornea. A femtosecond surgical laser 81 generates a pulsed laser beam 83 and directs that beam into the focusing assembly 85, which in turn focuses the pulsed beam 83 into the cornea 87. A contact lens 89 is placed over the cornea to deform the anterior corneal surface as described above. Where different contact lenses are used with the donor cornea and the recipient cornea, the posterior curvature, i.e., the side of the contact lens that is placed against the anterior corneal surface, is the same for each contact lens. The controller 91 is a programmable computer which precisely controls the location of the beam focal point within the cornea 87 according to parameters received from the surgeon interface 93. The interface 93 presents the surgeon with several incision patterns from which the desired sidecut pattern is selected. The selected pattern is received by the controller 91, which uses the pattern to incise both the donor cornea and the recipient cornea, placing the annular region of the selected pattern at a predetermined depth from each respective anterior corneal surface. The contact lenses which are used to deform each of the corneas as part of this procedure preferably have the same curvature on the posterior surface, i.e., the surface placed in contact with the anterior corneal surface.

Thus, a method of transplanting corneal tissue is disclosed. While embodiments of this invention have been shown and described, it will be apparent to those skilled in the art that many more modifications are possible without departing from the scope of the following claims.

## Claims

1. A system for resecting corneal tissue, the system comprising:
a surgical laser (11) adapted to emit a pulsed laser beam;
a focusing assembly adapted to focus the pulsed laser beam into a cornea;
an interface (93) adapted to provide a plurality of incision patterns for selection of a sidecut pattern which includes an annular region; and
a controller (91) in communication with the interface to receive the sidecut pattern, the controller (91) being adapted to move a focal point of the pulsed laser beam within a cornea (11) using the focusing assembly, to direct the focal point of the pulsed laser beam to make a first sidecut incision in the cornea according to the sidecut pattern, wherein the annular region of the sidecut pattern is incised at a predetermined second incision depth from an anterior corneal surface and the first sidecut incision runs from an outer periphery of the annular incision toward one of the anterior corneal surface and a posterior corneal surface, and to make a second sidecut incision wherein the second sidecut incision runs from an inner periphery of the annular incision toward the other of the anterior corneal surface and the posterior corneal surface, wherein a combination of the incisions in the donor cornea resects corneal tissue from the donor cornea, wherein the first sidecut region forms a first acute angle with the annular region and/or the second sidecut region forms a second acute angle with the annular region;
wherein the controller (91) is adapted to move a focal point of the pulsed laser beam within the cornea using the focusing assembly to direct the focal point of the pulsed laser beam to make a resection incision (51) at a predetermined first incision depth from an anterior corneal surface, wherein the second incision depth is less than the first incision depth, to make the first sidecut incision (59) so that the first sidecut incision runs from the outer periphery of the annular incision ( 55) to one of the anterior corneal surface and the resection incision (51), and to make the second sidecut incision ( 61) so that the second sidecut incision (61) runs from the inner periphery of the annular incision (55) to the other of the anterior corneal surface and the resection incision (51); or
wherein the controller (91) is adapted to move a focal point of the pulsed laser beam within the cornea using the focusing assembly to direct the focal point of the pulsed laser beam to make a resection incision (63) in the donor cornea at a first incision depth from the anterior corneal surface wherein the second incision depth is greater than the first incision depth, to make the first sidecut incision (71) so that the first sidecut incision (71) runs from an outer periphery of the annular incision (67) to one of the posterior corneal surface and the resection incision (63), and to make the second sidecut incision (73) so that the second sidecut incision (73) runs from the inner periphery of the annular incision (67) to the other of the posterior corneal surface and the resection incision (63).

2. The system of claim 1 further comprising: a contact lens (13) adapted to be placed against the anterior corneal surface, the contact lens (13) being adapted to conform the anterior corneal surface to a shape of the contact lens, optionally wherein the contact lens (13) applanates the anterior corneal surface.

3. The system of any preceding claim, further comprising an optical coherence tomography scanner to determine a thickness profile of the cornea.

4. The system of any preceding claim, wherein the annular incision is at a uniform distance from the anterior corneal surface.

5. The system of any preceding claim, wherein the controller (91) is adapted to move a focal point of the pulsed laser beam within the cornea using the focusing assembly to direct the focal point of the pulsed laser beam to make the resection incision (51) at the predetermined first incision depth from the anterior corneal surface, wherein the second incision depth is less than the first incision depth, to make the first sidecut incision (59) so that the first sidecut incision runs from the outer periphery of the annular incision (55) to one of the anterior corneal surface and the resection incision (51), and to make the second sidecut incision (61) so that the second sidecut incision (61) runs from the inner periphery of the annular incision (55) to the other of the anterior corneal surface and the resection incision ( 51 ), wherein the resection incision is at a uniform distance from the anterior corneal surface.

6. The system of any of claims 1 to 3, wherein the controller (91) is adapted to move a focal point of the pulsed laser beam within the cornea using the focusing assembly to direct the focal point of the pulsed laser beam to make the resection incision (63) in the donor cornea at the first incision depth from the anterior corneal surface wherein the second incision depth is greater than the first incision depth, to make the first sidecut incision (71) so that the first sidecut incision (71) runs from an outer periphery of the annular incision (67) to one of the posterior corneal surface and the resection incision (63), and to make the second sidecut incision (73) so that the second sidecut incision (73) runs from the inner periphery of the annular incision (67) to the other of the posterior corneal surface and the resection incision (63), wherein:
the annular incision is at a uniform distance from the posterior corneal surface; and/ or
the resection incision is at a uniform distance from the posterior corneal surface.

7. A method of resecting donor tissue from a donor cornea for transplant in a recipient cornea, the method comprising:
determining an incision depth from an anterior corneal surface of the donor cornea;
making an annular incision at the incision depth in the donor cornea, wherein the incision depth is a second incision depth;
making a first sidecut incision in the donor cornea, the first sidecut incision running from an outer periphery of the annular incision toward one of the anterior corneal surface and a posterior corneal surface; and
making a second sidecut incision in the donor cornea, the second sidecut incision running from an inner periphery of the annular incision toward the other of the anterior corneal surface and the posterior corneal surface, wherein a combination of the incisions in the donor cornea resects corneal tissue from the donor cornea, wherein the first sidecut region forms a first acute angle with the annular region and/or the second sidecut region forms a second acute angle with the annular region;
wherein the method further comprises determining a first incision depth from an anterior corneal surface, wherein the second incision depth is less than the first incision depth, making a resection incision (51) at the first incision depth in the donor cornea, making the first sidecut incision (59) so that the first sidecut incision (59) runs from the outer periphery of the annular incision (55) to one of the anterior corneal surface and the resection incision (51), and making the second sidecut incision (61) so that the second sidecut incision (61) runs from the inner periphery of the annular incision (55) to the other of the anterior corneal surface and the resection incision (51); or
wherein the method further comprises determining a first incision depth from an anterior corneal surface, wherein the second incision depth is greater than the first incision depth, making a resection incision (63) at the first incision depth in the donor cornea, making the first sidecut incision (71) so that the first sidecut incision (71) runs from an outer periphery of the annular incision (67) to one of the posterior corneal surface and the resection incision (63), and making the second sidecut incision (73) so that the second sidecut incision runs from the inner periphery of the annular incision (67) to the other of the posterior corneal surface and the resection incision (63).

8. The method of claim 7, further comprising determining a thickness profile of the cornea using an optical coherence tomography scanner.

9. The method of claim 7 or 8, further comprising placing a contact lens (13) against the anterior corneal surface of the donor cornea prior to making the incisions in the cornea, wherein the contact lens is adapted to conform the anterior corneal surface to a shape of the contact lens, optionally wherein the contact lens applanates the anterior corneal surface.

10. The method of any of claims 7 to 9, wherein the annular incision is at a uniform distance from the anterior corneal surface.

11. The method of any of claims 7 to 10, wherein the method comprises:
determining the first incision depth from the anterior corneal surface, wherein the second incision depth is less than the first incision depth;
making the resection incision (51) at the first incision depth in the donor cornea;
making the first sidecut incision (59) so that the first sidecut incision (59) runs from the outer periphery of the annular incision to one of the anterior corneal surface and the resection incision (51); and
making the second sidecut incision (61) so that the second sidecut incision runs from the inner periphery of the annular incision to the other of the anterior corneal surface and the resection incision (51), wherein the resection incision is at a uniform distance from the anterior corneal surface.

12. The method of any of claims 7 to 9, wherein the method comprises:
determining the first incision depth from the anterior corneal surface, wherein the second incision depth is greater than the first incision depth;
making the resection incision (63) at the first incision depth in the donor cornea;
making the first sidecut incision (71) so that the first sidecut incision (71) runs from an outer periphery of the annular incision to one of the posterior corneal surface and the resection incision (63); and
making the second sidecut incision (73) so that the second sidecut incision (73) runs from the inner periphery of the annular incision (67) to the other of the posterior corneal surface and the resection incision (63), wherein the annular incision is at a uniform distance from the posterior corneal surface and/ or wherein the resection incision is at a uniform distance from the posterior corneal surface.

## Patentansprüche

1. System zur Hornhautgeweberesektion, wobei das System umfasst:
einen Chirurgielaser (11), der dafür geeignet ist, einen gepulsten Laserstrahl auszusenden;
eine Fokussiereinrichtung, die dafür geeignet ist, den gepulsten Laserstrahl in eine Hornhaut zu fokussieren;
eine Schnittstelle (93), die dafür geeignet ist, eine Vielzahl von Schnittführungen für die Auswahl einer seitlichen Schnittführung, die eine ringförmige Region enthält, bereitzustellen; und
eine Steuereinheit (91) in Kommunikation mit der Schnittstelle zum Empfangen der seitlichen Schnittführung, wobei die Steuereinheit (91) dafür geeignet ist, einen Fokus des gepulsten Laserstrahls in einer Hornhaut (11) mittels der Fokussiereinrichtung zu verschieben, um den Fokus des gepulsten Laserstrahls zu richten, um einen ersten seitlichen Schnitt in die Hornhaut nach der seitlichen Schnittführung durchzuführen, wobei die ringförmige Region der seitlichen Schnittführung in einer vorbestimmten zweiten Schnitttiefe von einer vorderen Hornhautoberfläche eingeschnitten wird und der erste seitliche Schnitt von einem ersten Umfang des ringförmigen Schnitts zu einer von der vorderen Hornhautoberfläche und einer hinteren Hornhautoberfläche verläuft, und um einen zweiten seitlichen Schnitt durchzuführen, wobei der zweite seitliche Schnitt von einem inneren Umfang des ringförmigen Schnitts zur anderen von der vorderen Hornhautoberfläche und der hinteren Hornhautoberfläche verläuft, wobei eine Kombination der Schnitte in die Spender-Hornhaut Hornhautgewebe von der Spender-Hornhaut reseziert, wobei die Region des ersten seitlichen Schnitts einen ersten spitzen Winkel zur ringförmigen Region bildet und/oder die Region des zweiten seitlichen Schnitts einen zweiten spitzen Winkel zur ringförmigen Region bildet;
wobei die Steuereinheit (91) dafür geeignet ist, einen Fokus des gepulsten Laserstrahls in der Hornhaut mittels der Fokussiereinrichtung zu bewegen, um den Fokus des gepulsten Laserstrahls zu richten, um einen Resektionsschnitt (51) in einer vorbestimmten ersten Schnitttiefe von einer vorderen Hornhautoberfläche durchzuführen, wobei die zweite Schnitttiefe geringer als die erste Schnitttiefe ist, den ersten seitlichen Schnitt (59) derart durchzuführen, dass der erste seitliche Schnitt vom äußeren Umfang des ringförmigen Schnitts (55) zu einem von der vorderen Hornhautoberfläche und dem Resektionsschnitt (51) verläuft, und den zweiten seitlichen Schnitt (61) derart durchzuführen, dass der zweite seitliche Schnitt (61) vom inneren Umfang des ringförmigen Schnitts (55) zum anderen von der vorderen Hornhautoberfläche und dem Resektionsschnitt (51) verläuft; oder
wobei die Steuereinheit (91) dafür geeignet ist, einen Fokus des gepulsten Laserstrahls in der Hornhaut mittels der Fokussiereinrichtung zu bewegen, um den Fokus des gepulsten Laserstrahls zu richten, um einen Resektionsschnitt (63) in die Spender-Hornhaut in einer ersten Schnitttiefe von der vorderen Hornhautoberfläche durchzuführen, wobei die zweite Schnitttiefe größer als die erste Schnitttiefe ist, den ersten seitlichen Schnitt (71) derart durchzuführen, dass der erste seitliche Schnitt (71) von einem äußeren Umfang des ringförmigen Schnitts (67) zu einem von der hinteren Hornhautoberfläche und dem Resektionsschnitt (63) verläuft, und den zweiten seitlichen Schnitt (73) derart durchzuführen, dass der zweite seitliche Schnitt (73) vom inneren Umfang des ringförmigen Schnitts (67) zum anderen von der hinteren Hornhautoberfläche und dem Resektionsschnitt (63) verläuft.

2. System nach Anspruch 1, ferner umfassend: eine Kontaktlinse (13), die dafür geeignet ist, auf die vordere Hornhautoberfläche gelegt zu werden, wobei die Kontaktlinse (13) dafür geeignet ist, die vordere Hornhautoberfläche an eine Form der Kontaktlinse anzupassen, wobei wahlweise die Kontaktlinse (13) die vordere Hornhautoberfläche applaniert.

3. System nach einem der vorhergehenden Ansprüche, das ferner einen optischen Kohärenztomographen umfasst, um ein Dickenprofil der Hornhaut zu bestimmen.

4. System nach einem der vorhergehenden Ansprüche, wobei der ringförmige Schnitt in einem einheitlichen Abstand von der vorderen Hornhautoberfläche liegt.

5. System nach einem der vorhergehenden Ansprüche, wobei die Steuereinheit (91) dafür geeignet ist, einen Fokus des gepulsten Laserstrahl in der Hornhaut mittels der Fokussiereinrichtung zu bewegen, um den Fokus des gepulsten Laserstrahls zu richten, um den Resektionsschnitt (51) in der vorbestimmten ersten Schnitttiefe von der vorderen Hornhautoberfläche durchzuführen, wobei die zweite Schnitttiefe geringer als die erste Schnitttiefe ist, den ersten seitlichen Schnitt (59) derart durchzuführen, dass der erste seitliche Schnitt vom äußeren Umfang des ringförmigen Schnitts (55) zu einem von der vorderen Hornhautoberfläche und dem Resektionsschnitt (51) verläuft, und den zweiten seitlichen Schnitt (61) derart durchzuführen, dass der zweite seitliche Schnitt (61) vom inneren Umfang des ringförmigen Schnitts (55) zum anderen von der vorderen Hornhautoberfläche und dem Resektionsschnitt (51) verläuft, wobei der Resektionsschnitt in einem einheitlichen Abstand von der vorderen Hornhautoberfläche liegt.

6. System nach einem der Ansprüche 1 bis 3, wobei die Steuereinheit (91) dafür geeignet ist, einen Fokus des gepulsten Laserstrahls in der Hornhaut mittels der Fokussiereinrichtung zu bewegen, um den Fokus des gepulsten Laserstrahls zu richten, um den Resektionsschnitt (63) in die Spender-Hornhaut mit der ersten Schnitttiefe von der vorderen Hornhautoberfläche durchzuführen, wobei die zweite Schnitttiefe größer als die erste Schnitttiefe ist, den ersten seitlichen Schnitt (71) derart durchzuführen, dass der erste seitliche Schnitt (71) von einem äußeren Umfang des ringförmigen Schnitts (67) zu einem von der hinteren Hornhautoberfläche und dem Resektionsschnitt (63) verläuft, und den zweiten seitlichen Schnitt (73) derart durchzuführen, dass der zweite seitliche Schnitt (73) vom inneren Umfang des ringförmigen Schnitts (67) zum anderen von der hinteren Hornhautoberfläche und dem Resektionsschnitt (63) verläuft, wobei:
der ringförmige Schnitt in einem einheitlichen Abstand von der hinteren Hornhautoberfläche liegt; und/oder
der Resektionsschnitt in einem einheitlichen Abstand von der hinteren Hornhautoberfläche liegt.

7. Verfahren zur Resektion von Spender-Hornhautgewebe von einer Spender-Hornhaut zum Transplantieren auf eine Empfänger-Hornhaut, wobei das Verfahren umfasst:
Bestimmen einer Schnitttiefe von einer vorderen Hornhautoberfläche der Spender-Hornhaut;
Durchführen eines ringförmigen Schnitts mit der Schnitttiefe in die Spender-Hornhaut, wobei die Schnitttiefe eine zweite Schnitttiefe ist;
Durchführen eines ersten seitlichen Schnitts in die Spender-Hornhaut, wobei der erste seitliche Schnitt von einem äußeren Umfang des ringförmigen Schnitts zu einer von der vorderen Hornhautoberflache und einer hinteren Hornhautoberfläche verläuft; und
Durchführen eines zweiten seitlichen Schnitts in die Spender-Hornhaut, wobei der zweite seitliche Schnitt von einem inneren Umfang des ringförmigen Schnitts zur anderen von der vorderen Hornhautoberfläche und der hinteren Hornhautoberfläche verläuft, wobei eine Kombination der Schnitte in die Spender-Hornhaut Hornhautgewebe von der Spender-Hornhaut reseziert, wobei die Region des ersten seitlichen Schnitts einen ersten spitzen Winkel zur ringförmigen Region bildet und/oder die Region des zweiten seitlichen Schnitts einen zweiten spitzen Winkel zur ringförmigen Region bildet;
wobei das Verfahren ferner umfasst: Bestimmen einer ersten Schnitttiefe von einer vorderen Hornhautoberfläche, wobei die zweite Schnitttiefe geringer als die erste Schnitttiefe ist, Durchführen eines Resektionsschnitts (51) mit der ersten Schnitttiefe in die Spender-Hornhaut, Durchführen des ersten seitlichen Schnitts (59) derart, dass der erste seitliche Schnitt (59) vom äußeren Umfang des ringförmigen Schnitts (55) zu einem von der vorderen Hornhautoberfläche und dem Resektionsschnitt (51) verläuft, und Durchführen des zweiten seitlichen Schnitts (61) derart, dass der zweite seitliche Schnitt (61) vom inneren Umfang des ringförmigen Schnitts (55) zum anderen von der vorderen Hornhautoberfläche und dem Resektionsschnitt (51) verläuft; oder
wobei das Verfahren ferner umfasst: Bestimmen einer ersten Schnitttiefe von einer vorderen Hornhautoberfläche, wobei die zweite Schnitttiefe größer als die erste Schnitttiefe ist, Durchführen eines Resektionsschnitts (63) mit der ersten Schnitttiefe in die Spender-Hornhaut, Durchführen des ersten seitlichen Schnitts (71) derart, dass der erste seitliche Schnitt (71) von einem äußeren Umfang des ringförmigen Schnitts (67) zu einem von der hintern Hornhautoberfläche und dem Resektionsschnitt (63) verläuft, und Durchführen des zweiten seitlichen Schnitts (73) derart, dass der zweite seitliche Schnitt vom inneren Umfang des ringförmigen Schnitts (67) zum anderen von der hinteren Hornhautoberfläche und dem Resektionsschnitt (63) verläuft.

8. Verfahren nach Anspruch 7, das ferner Bestimmen eines Dickenprofils der Hornhaut mittels eines optischen Kohärenztomographen umfasst.

9. Verfahren nach Anspruch 7 oder 8, das ferner Auflegen einer Kontaktlinse (13) auf die vordere Hornhautoberfläche der Spender-Hornhaut vor dem Durchführen der Schnitte in die Hornhaut umfasst, wobei die Kontaktlinse dafür geeignet ist, die vordere Hornhautoberfläche an eine Form der Kontaktlinse anzupassen, wobei wahlweise die Kontaktlinse die vordere Hornhautoberfläche applaniert.

10. Verfahren nach einem der Ansprüche 7 bis 9, wobei der ringförmige Schnitt in einem einheitlichen Abstand von der vorderen Hornhautoberfläche liegt.

11. Verfahren nach einem der Ansprüche 7 bis 10, wobei das Verfahren umfasst:
Bestimmen der ersten Schnitttiefe von der vorderen Hornhautoberfläche, wobei die zweite Schnitttiefe geringer als die erste Schnitttiefe ist;
Durchführen des Resektionsschnitts (51) mit der ersten Schnitttiefe in die Spender-Hornhaut;
Durchführen des ersten seitlichen Schnitts (59) derart, dass der erste seitliche Schnitt (59) vom äußeren Umfang des ringförmigen Schnitts zu einem von der vorderen Hornhautoberfläche und dem Resektionsschnitt (51) verläuft; und
Durchführen des zweiten seitlichen Schnitts (61) derart, dass der zweite seitliche Schnitt vom inneren Umfang des ringförmigen Schnitts zum anderen von der vorderen Hornhautoberfläche und dem Resektionsschnitt (51) verläuft, wobei der Resektionsschnitt in einem einheitlichen Abstand von der vorderen Hornhautoberfläche liegt.

12. Verfahren nach einem der Ansprüche 7 bis 9, wobei das Verfahren umfasst:
Bestimmen der ersten Schnitttiefe von der vorderen Hornhautoberfläche, wobei die zweite Schnitttiefe größer als die erste Schnitttiefe ist;
Durchführen des Resektionsschnitts (63) mit der ersten Schnitttiefe in die Spender-Hornhaut;
Durchführen des ersten seitlichen Schnitts (71) derart, dass der erste seitliche Schnitt (71) von einem äußeren Umfang des ringförmigen Schnitts zu einer von einer hinteren Hornhautoberfläche und dem Resektionsschnitt (63) verläuft; und
Durchführen des zweiten seitlichen Schnitts (73) derart, dass der zweite seitliche Schnitt (73) vom inneren Umfang des ringförmigen Schnitts (67) zum anderen von der hinteren Hornhautoberfläche und dem Resektionsschnitt (63) verläuft, wobei der ringförmige Schnitt in einem einheitlichen Abstand von der hinteren Hornhautoberfläche liegt und/oder wobei der Resektionsschnitt in einem einheitlichen Abstand von der hinteren Hornhautoberfläche liegt.

## Revendications

1. Système de résection de tissu cornéen, le système comprenant :
un laser chirurgical (11) adapté pour émettre un faisceau laser pulsé ;
un ensemble de focalisation adapté pour focaliser le faisceau laser pulsé à l'intérieur d'une cornée ;
une interface (93) adaptée pour fournir une pluralité de motifs d'incision pour sélection d'un motif de coupe latérale qui comporte une région annulaire ; et
un contrôleur (91) en communication avec l'interface pour recevoir le motif de coupe latérale, le contrôleur (91) étant adapté pour déplacer un foyer du faisceau laser pulsé à l'intérieur d'une cornée (11) en utilisant l'ensemble de focalisation, pour orienter le foyer du faisceau laser pulsé pour pratiquer une première incision latérale dans la cornée en fonction du motif de coupe latérale, la région annulaire du motif de coupe latérale étant incisée à une deuxième profondeur d'incision prédéterminée depuis une surface cornéenne antérieure et la première incision latérale partant d'une périphérie externe de l'incision annulaire vers une surface parmi la surface cornéenne antérieure et une surface cornéenne postérieure, et pour pratiquer une deuxième incision latérale, la deuxième incision latérale partant d'une périphérie interne de l'incision annulaire vers l'autre surface parmi la surface cornéenne antérieure et la surface cornéenne postérieure, une combinaison des incisions dans la cornée de donneur assurant la résection de tissu cornéen depuis la cornée de donneur, la première région de coupe latérale formant un premier angle aigu avec la région annulaire et/ou la deuxième région de coupe latérale formant un deuxième angle aigu avec la région annulaire ;
dans lequel le contrôleur (91) est adapté pour déplacer un foyer du faisceau laser pulsé à l'intérieur de la cornée en utilisant l'ensemble de focalisation pour orienter le foyer du faisceau laser pulsé pour pratiquer une incision de résection (51) à une première profondeur d'incision prédéterminée depuis une surface cornéenne antérieure, la deuxième profondeur d'incision étant inférieure à la première profondeur d'incision, pour pratiquer la première incision latérale (59) de telle sorte que la première incision latérale part de la périphérie externe de l'incision annulaire (55) jusqu'à un emplacement parmi la surface cornéenne antérieure et l'incision de résection (51), et pour pratiquer la deuxième incision latérale (61) de telle sorte que la deuxième incision latérale (61) part de la périphérie interne de l'incision annulaire (55) jusqu'à l'autre emplacement parmi la surface cornéenne antérieure et l'incision de résection (51) ; ou
dans lequel le contrôleur (91) est adapté pour déplacer un foyer du faisceau laser pulsé à l'intérieur de la cornée en utilisant l'ensemble de focalisation pour orienter le foyer du faisceau laser pulsé pour pratiquer une incision de résection (63) dans la cornée de donneur à une première profondeur d'incision depuis la surface cornéenne antérieure, la deuxième profondeur d'incision étant supérieure à la première profondeur d'incision, pour pratiquer la première incision latérale (71) de telle sorte que la première incision latérale (71) part d'une périphérie externe de l'incision annulaire (67) jusqu'à un emplacement parmi la surface cornéenne postérieure et l'incision de résection (63), et pour pratiquer la deuxième incision latérale (73) de telle sorte que la deuxième incision latérale (73) part de la périphérie interne de l'incision annulaire (67) jusqu'à l'autre emplacement parmi la surface cornéenne postérieure et l'incision de résection (63).

2. Système de la revendication 1 comprenant en outre : une lentille de contact (13) adaptée pour être placée contre la surface cornéenne antérieure, la lentille de contact (13) étant adaptée pour que la surface cornéenne antérieure épouse une forme de la lentille de contact, éventuellement dans lequel la lentille de contact (13) aplanit la surface cornéenne antérieure.

3. Système d'une quelconque revendication précédente, comprenant en outre un tomodensitomètre à cohérence optique pour déterminer un profil d'épaisseur de la cornée.

4. Système d'une quelconque revendication précédente, dans lequel l'incision annulaire est à une distance uniforme de la surface cornéenne antérieure.

5. Système d'une quelconque revendication précédente, dans lequel le contrôleur (91) est adapté pour déplacer un foyer du faisceau laser pulsé à l'intérieur de la cornée en utilisant l'ensemble de focalisation pour orienter le foyer du faisceau laser pulsé pour pratiquer l'incision de résection (51) à la première profondeur d'incision prédéterminée depuis la surface cornéenne antérieure, la deuxième profondeur d'incision étant inférieure à la première profondeur d'incision, pour pratiquer la première incision latérale (59) de telle sorte que la première incision latérale part de la périphérie externe de l'incision annulaire (55) jusqu'à un emplacement parmi la surface cornéenne antérieure et l'incision de résection (51), et pour pratiquer la deuxième incision latérale (61) de telle sorte que la deuxième incision latérale (61) part de la périphérie interne de l'incision annulaire (55) jusqu'à l'autre emplacement parmi la surface cornéenne antérieure et l'incision de résection (51), dans lequel l'incision de résection est à une distance uniforme de la surface cornéenne antérieure.

6. Système de l'une quelconque des revendications 1 à 3, dans lequel le contrôleur (91) est adapté pour déplacer un foyer du faisceau laser pulsé à l'intérieur de la cornée en utilisant l'ensemble de focalisation pour orienter le foyer du faisceau laser pulsé pour pratiquer l'incision de résection (63) dans la cornée de donneur à la première profondeur d'incision depuis la surface cornéenne antérieure, la deuxième profondeur d'incision étant supérieure à la première profondeur d'incision, pour pratiquer la première incision latérale (71) de telle sorte que la première incision latérale (71) part de la périphérie externe de l'incision annulaire (67) jusqu'à un emplacement parmi la surface cornéenne postérieure et l'incision de résection (63), et pour pratiquer la deuxième incision latérale (73) de telle sorte que la deuxième incision latérale (73) part de la périphérie interne de l'incision annulaire (67) jusqu'à l'autre emplacement parmi la surface cornéenne postérieure et l'incision de résection (63), dans lequel :
l'incision annulaire est à une distance uniforme de la surface cornéenne postérieure ; et/ou
l'incision de résection est à une distance uniforme de la surface cornéenne postérieure.

7. Procédé de résection de tissu de donneur depuis une cornée de donneur pour transplantation dans une cornée de receveur, le procédé comprenant :
la détermination d'une profondeur d'incision depuis une surface cornéenne antérieure de la cornée de donneur ;
la pratique d'une incision annulaire à la profondeur d'incision dans la cornée de donneur, la profondeur d'incision étant une deuxième profondeur d'incision ;
la pratique d'une première incision latérale dans la cornée de donneur, la première incision latérale partant d'une périphérie externe de l'incision annulaire vers une surface parmi la surface cornéenne antérieure et une surface cornéenne postérieure ; et
la pratique d'une deuxième incision latérale dans la cornée de donneur, la deuxième incision latérale partant d'une périphérie interne de l'incision annulaire vers l'autre surface parmi la surface cornéenne antérieure et la surface cornéenne postérieure, une combinaison des incisions dans la cornée de donneur assurant la résection de tissu cornéen depuis la cornée de donneur, la première région de coupe latérale formant un premier angle aigu avec la région annulaire et/ou la deuxième région de coupe latérale formant un deuxième angle aigu avec la région annulaire ;
le procédé comprenant en outre la détermination d'une première profondeur d'incision depuis une surface cornéenne antérieure, la deuxième profondeur d'incision étant inférieure à la première profondeur d'incision, la pratique d'une incision de résection (51) à la première profondeur d'incision dans la cornée de donneur, la pratique de la première incision latérale (59) de telle sorte que la première incision latérale (59) parte de la périphérie externe de l'incision annulaire (55) jusqu'à un emplacement parmi la surface cornéenne antérieure et l'incision de résection (51), et la pratique de la deuxième incision latérale (61) de telle sorte que la deuxième incision latérale (61) parte de la périphérie interne de l'incision annulaire (55) jusqu'à l'autre emplacement parmi la surface cornéenne antérieure et l'incision de résection (51) ; ou
le procédé comprenant en outre la détermination d'une première profondeur d'incision depuis une surface cornéenne antérieure, la deuxième profondeur d'incision étant supérieure à la première profondeur d'incision, la pratique d'une incision de résection (63) à la première profondeur d'incision dans la cornée de donneur, la pratique de la première incision latérale (71) de telle sorte que la première incision latérale (71) parte d'une périphérie externe de l'incision annulaire (67) jusqu'à un emplacement parmi la surface cornéenne postérieure et l'incision de résection (63), et la pratique de la deuxième incision latérale (73) de telle sorte que la deuxième incision latérale parte de la périphérie interne de l'incision annulaire (67) jusqu'à l'autre emplacement parmi la surface cornéenne postérieure et l'incision de résection (63).

8. Procédé de la revendication 7, comprenant en outre la détermination d'un profil d'épaisseur de la cornée au moyen d'un tomodensitomètre à cohérence optique.

9. Procédé de la revendication 7 ou 8, comprenant en outre la mise en place d'une lentille de contact (13) contre la surface cornéenne antérieure de la cornée de donneur avant la pratique des incisions dans la cornée, dans lequel la lentille de contact est adaptée pour que la surface cornéenne antérieure épouse une forme de la lentille de contact, éventuellement dans lequel la lentille de contact aplanit la surface cornéenne antérieure.

10. Procédé de l'une quelconque des revendications 7 à 9, dans lequel l'incision annulaire est à une distance uniforme de la surface cornéenne antérieure.

11. Procédé de l'une quelconque des revendications 7 à 10, le procédé comprenant :
la détermination de la première profondeur d'incision depuis la surface cornéenne antérieure, la deuxième profondeur d'incision étant inférieure à la première profondeur d'incision ;
la pratique de l'incision de résection (51) à la première profondeur d'incision dans la cornée de donneur ;
la pratique de la première incision latérale (59) de telle sorte que la première incision latérale (59) parte de la périphérie externe de l'incision annulaire jusqu'à un emplacement parmi la surface cornéenne antérieure et l'incision de résection (51) ; et
la pratique de la deuxième incision latérale (61) de telle sorte que la deuxième incision latérale parte de la périphérie interne de l'incision annulaire jusqu'à l'autre emplacement parmi la surface cornéenne antérieure et l'incision de résection (51), dans lequel l'incision de résection est à une distance uniforme de la surface cornéenne antérieure.

12. Procédé de l'une quelconque des revendications 7 à 9, le procédé comprenant :
la détermination de la première profondeur d'incision depuis la surface cornéenne antérieure, la deuxième profondeur d'incision étant supérieure à la première profondeur d'incision ;
la pratique de l'incision de résection (63) à la première profondeur d'incision dans la cornée de donneur ;
la pratique de la première incision latérale (71) de telle sorte que la première incision latérale (71) parte d'une périphérie externe de l'incision annulaire jusqu'à un emplacement parmi la surface cornéenne postérieure et l'incision de résection (63) ; et
la pratique de la deuxième incision latérale (73) de telle sorte que la deuxième incision latérale (73) parte de la périphérie interne de l'incision annulaire (67) jusqu'à l'autre emplacement parmi la surface cornéenne postérieure et l'incision de résection (63), dans lequel l'incision annulaire est à une distance uniforme de la surface cornéenne postérieure et/ou dans lequel l'incision de résection est à une distance uniforme de la surface cornéenne postérieure.
